(19) Europäisches Patentamt
European Patent Office
Office européen des brevets

(11) Publication number: 0 162 265
B1

(12) **EUROPEAN PATENT SPECIFICATION**

(45) Date of publication of patent specification: **03.10.90**

(51) Int. Cl.$^5$: **C 07 D 307/32** // C07D405/06, A01N43/50, A01N43/64

(21) Application number: **85104302.6**

(22) Date of filing: **26.03.82**

(60) Publication number of the earlier application in accordance with Art. 76 EPC: **0 061 910**

(54) **The preparation of substituted gamma butyrolactones useful as intermediates for making fungicidal imidazoles and triazoles.**

(30) Priority: **30.03.81 US 249250**

(43) Date of publication of application:
**27.11.85 Bulletin 85/48**

(45) Publication of the grant of the patent:
**03.10.90 Bulletin 90/40**

(84) Designated Contracting States:
**AT BE CH DE FR GB IT LI NL SE**

(56) References cited:
**FR-A-2 456 097**

**CHEMICAL ABSTRACS, vol. 93, no. 7, 18 Aug 1980, Columbus, OH (US); M.LARCHEVEQUE et al.: "A convenient synthesis of gamma-hydroxy and gamma-keto nitriles", p. 944, no. 71413t**

**The file contains technical information submitted after the application was filed and not included in this specification**

(73) Proprietor: **ROHM AND HAAS COMPANY**
**Independence Mall West**
**Philadelphia Pennsylvania 19105 (US)**

(72) Inventor: **Chan, Hak-Foon**
**524 Matterhorn Drive**
**Walnut Creek, CA 94958 (US)**

(74) Representative: **Angell, David Whilton et al**
**ROHM AND HAAS (UK) LTD. European Operations Patent Department Lennig House 2 Mason's Avenue**
**Croydon CR9 3NB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 162 265 B1

Courier Press, Leamington Spa, England.

## Description

This invention concerns the preparation of certain substituted *gamma* butyrolactones useful as intermediates in the provision of fungicidal substituted imidazoles and triazoles.

Our European Application EP—A—0 061 910, from which this application is divided and so the whole contents of which attention is hereby specifically directed, is concerned with substituted imidazoles and triazoles which are characterized by having a novel N-substituent which contains a substituted butyrolactone residue.

More precisely those fungicides are 1-H-imidazoles, 1-H-1,2,4-triazoles or 4-H-1,2,4-triazoles having an N-substituent of the formula:

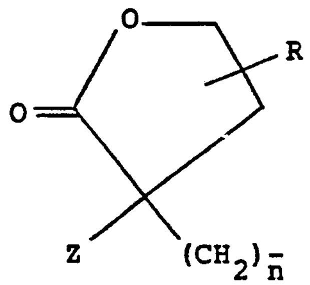

(I)

The compounds may be represented by the formula:

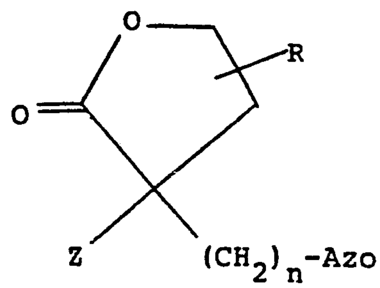

(II)

In Formula I and II above:

Z is phenyl or naphthyl (preferably phenyl) each optionally substituted with up to 3 (preferably 1 or 2) of the same or different substituents selected from (1) halo (viz chloro, bromo, fluoro or iodo), (2) nitro, (3) trihalomethyl (particularly $CF_3$), (4) cyano, (5) ($C_1$—$C_4$)alkyl, (6) ($C_1$—$C_4$)alkoxy, (7) ($C_1$—$C_4$)alkylthio (8) ($C_1$—$C_4$)alkylsulfinyl, (9) ($C_1$—$C_4$)alkylsulfonyl, (10) phenyl, (11) benzyl, (12) phenoxy, (13) phenylthio, (14) phenylsulfinyl, (15) phenylsulfonyl and (16) groups (10 to 15) above which contain up to 3 ring substituents selected from groups (1) to (9) above,

R is H, ($C_1$—$C_{10}$)alkyl, ($C_3$—$C_8$)alkenyl, ($C_3$—$C_8$)alkynyl, phenyl, naphthyl, phenyl($C_1$—$C_4$)alkyl, naphthyl($C_1$—$C_4$)alkyl or each of the last four groups containing up to 3, preferably 1 or 2, of the same or different ring substituents selected from groups (1) to (16) listed above in connection with the definition of Z,

n is zero or an integer of 1—5, and (in Formula II) Azo is 1-imidazolyl, 1-(1,2,4-triazolyl) or 4-(1,2,4-triazolyl).

Any alkyl, alkenyl or alkynyl groups in the compounds of Formula II may be straight or branched chain groups.

Also of interest are agronomically acceptable acid addition salts and agronomically acceptable metal salt complexes of compounds of Formula II.

Preferred compounds of Formula II are those wherein z is phenyl or phenyl substituted with up to two halogen atoms R is ($C_1$ to $C_4$)alkyl Azo is a 1-(imidazolyl) or 1-(1,2,4-triazolyl) and n is zero or the integer 1.

More preferred compounds of Formula II are those wherein Z is phenyl or 2,4-dichlorophenyl R is methyl or ethyl Azo is 1-(imidazolyl) or 1-(1,2,4-triazolyl) and n is zero or the integer 1.

Typical compounds of Formula II include:

4-ethyl-2-(1-imidazolyl)-2-(4-chlorophenyl)-*gamma*-butyrolactone;
4-methyl-2-(1-imidazolyl)-2-(2-chloro-4-fluorophenyl)-*gamma*-butyrolactone;
4-isopropyl-2-(1-imidazolyl)-2-(3,4-dibromophenyl)-*gamma*-butyrolactone;
4-*n*-propyl-2-(1-imidazolyl)-2-(3,5-diiodophenyl)-*gamma*-butyrolactone;
4-*n*-butyl-2-(1-imidazolyl)-2-(2,3-dinitrophenyl)-*gamma*-butyrolactone;
4-*n*-hexyl-2-(1-imidazolyl)-2-(4-trifluoromethylphenyl)-*gamma*-butyrolactone;
4-*iso*-heptyl-2-[1-(1,2,4-triazolyl)]-2-(2-chloro-4-cyanophenyl)-*gamma*-butyrolactone;
4-*n*-octyl-2-[1-(imidazolyl)methyl]-2-(3-cyanophenyl)-*gamma*-butyrolactone;
4-*sec*-nonyl-2-[1-(1,2,4-triazolyl)*n*-propyl]-2-(4-chloro-2-methylphenyl)-*gamma*-butyrolactone;

4-*n*-decyl-2-(1-imidazolyl)-2-(2,3,5-trimethylphenyl)-*gamma*-butyrolactone;
toluene. To the combined toluene extracts is added 50 ml of 50% by weight hydrochloric acid and the mixture
4-(2-octenyl)-2-(1-imidazolyl)-2-tolylphenyl-*gamma*-butyrolactone;
4-propargyl-2-([1-(1,2,4-triazolyl)*n*-pentyl]-2-(4-anisylphenyl)-*gamma*-butyrolactone;
4-(2-octynyl)-2-[1-(1,2,4-triazolyl)]-2-(4-methylthiophenyl)-*gamma*-butyrolactone;
4-benzyl-2-[1-(imidazolyl)methyl]-2-(4-methylsulfinylphenyl)-*gamma*-butyrolactone;
4-phenethyl-2-[1-(1,2,4-triazolyl)]-2-(4-methylsulfonylphenyl)-*gamma*-butyrolactone;
and the agronomically acceptable acid addition salts and metal salt complexes thereof.

For the preparation of compounds of Formula II wherein n = 1 to 5, reaction schemes A, B and C below may be used. A substituted *gamma*-butyrolactone (III) is reacted with a terminal dihaloalkane, e.g. dibromomethane or 1,3-dibromopropane, under strongly basic conditions which gives a 2-haloalkyl *gamma*-butyrolactone (IV). The reaction of (IV) with 1-*H*-imidazole (or metal (M) salt thereof), 1-*H*-1,2,4-triazole (or metal salt thereof) provides the expected product (reaction schemes B and C). When 1-*H*-1,2,4-triazole or salt thereof is used in this reaction, a mixture of the 1- and 4-substituted 1,2,4-triazolyl product (V and VI) is obtained as shown in reaction scheme C.

**Reaction Scheme A**

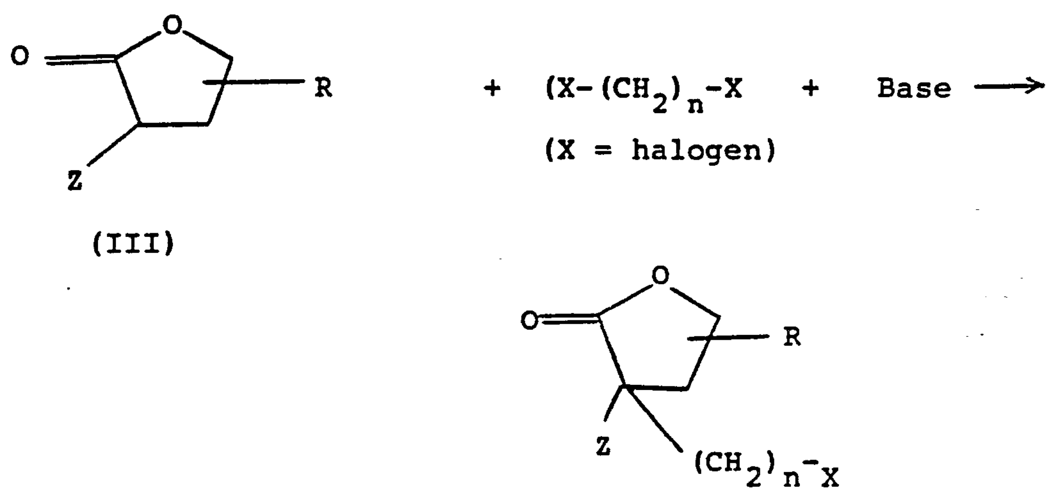


(III)

(IV) n=1-5

**Reaction Scheme B**

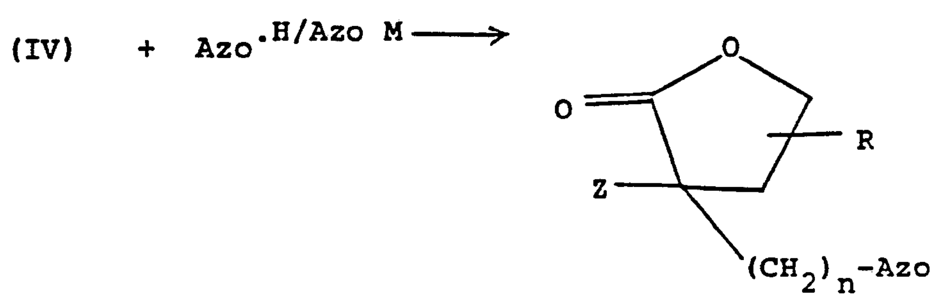


(n=1-5)

Reaction Scheme C

(IV) +

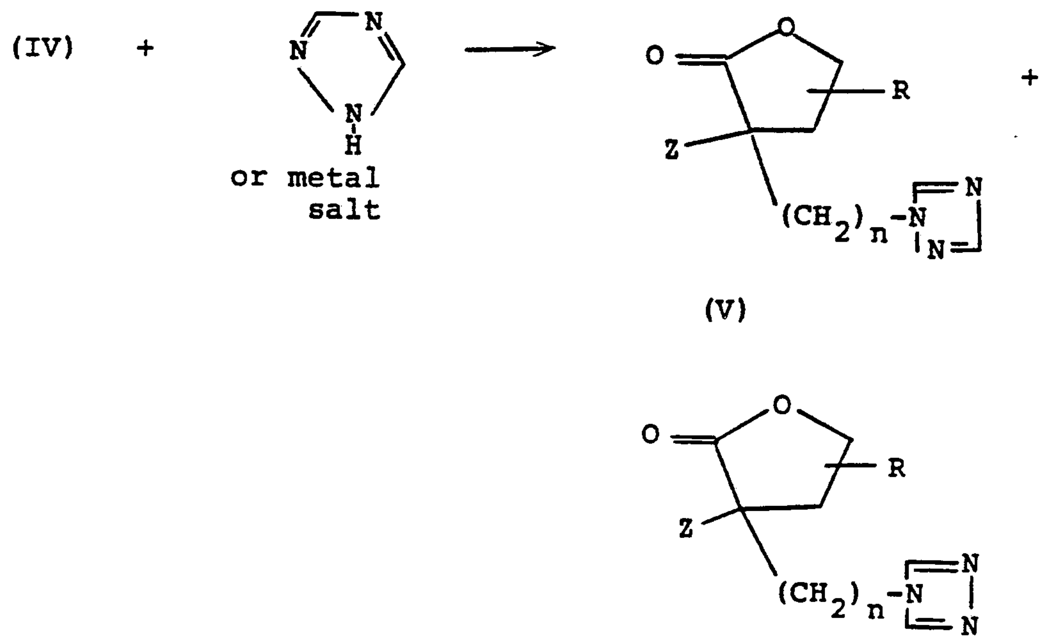


(V)

+

(VI)

Only a few substituted *gamma*-butyrolactones (III) are known in the literature and their synthesis involves tedious and low yield reactions. As described in Res. Commun. Chem. Pathol. Pharmacol., 1976, 15(1), 21—30, 2-phenyl-gamma-butyrolactone (VII), a metabolite of glutethimide, pheobarbital and pyrimidone in human urine, was synthesized in a 37% overall yield starting with diethylphenylmalonate (VIII), sodium hydride, and 1,2-dibromoethane, (reaction Scheme D below).

Reaction Scheme D

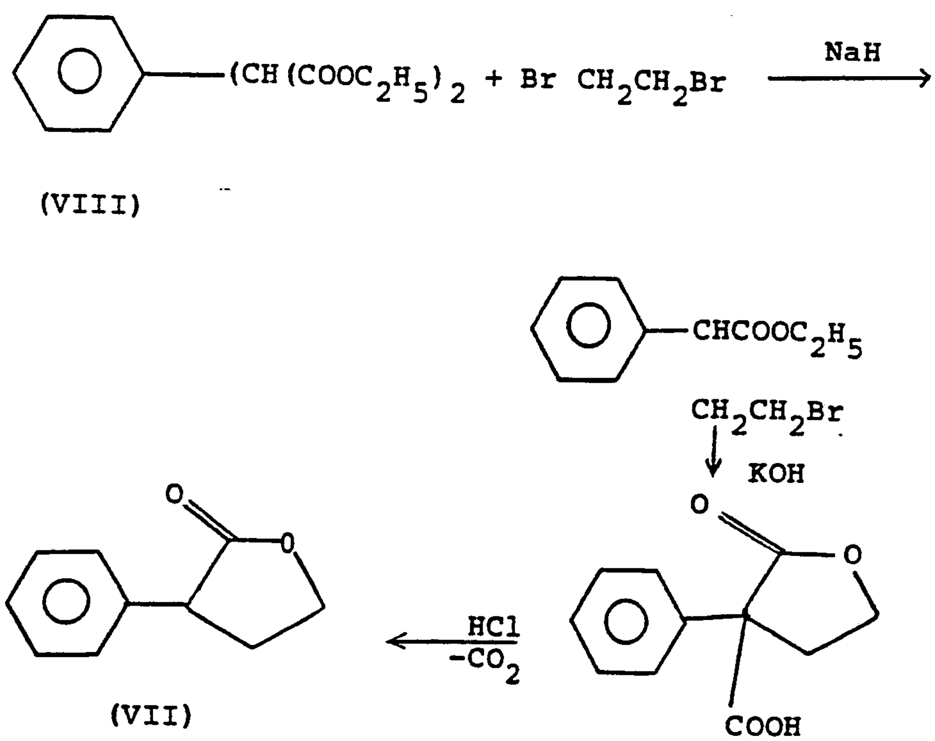


(VIII)

(VII)

We have now found that the *gamma*-butyrolactone of structure (III) may be prepared from an aryl acetonitrile (IX) and an epoxide (X). Alkylation of the aryl acetonitrile (IX) with an epoxide (X) provides a hydroxy cyanide (XI) which under basic conditions cyclizes to *gamma*-iminobutyrolactone (XII). Compound (XII) may then be hydrolyzed to the desired lactone product (III). These reactions are shown in reaction schemes E and F below.

Reaction Scheme E

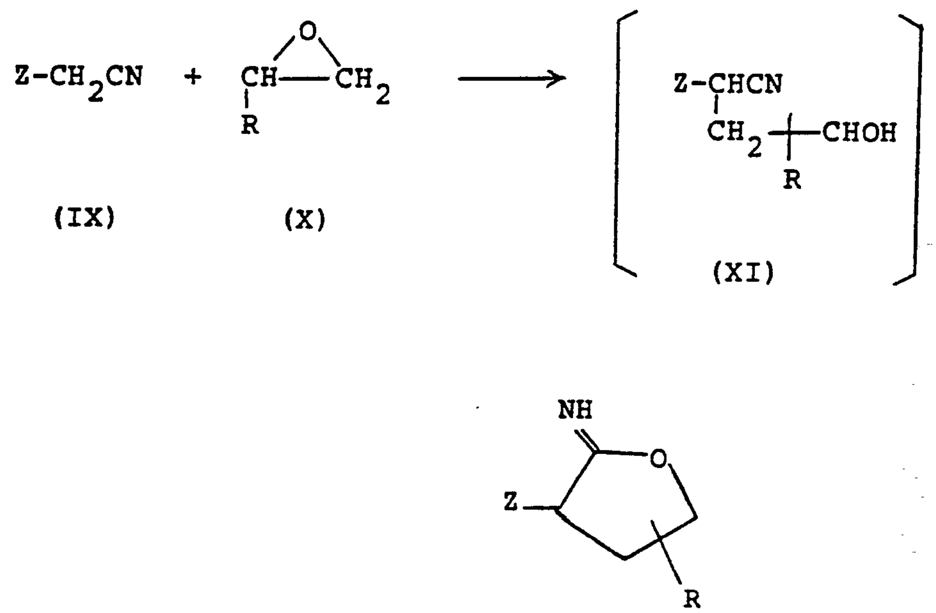

(IX) (X) (XI)

(XII)

Reaction Scheme F

(XII) 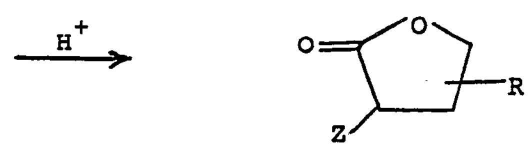


(III)

In the Examples given hereinafter, this process is carried out at from 5°C to room temperature, the basic conditions derive from the use of potassium hydroxide and dimethylformamide is used as organic solvent. This process can be contrasted with that of Synthetic Communications *10*(1) pp 49—57, 1980 wherein, using an alkylacetonitrile starting material, a temperature of −78°C is recommended.

Compounds of Formula II wherein n = 0, can also be prepared from a *gamma*-butyrolactone (III). Bromination of *gamma*-butyrolactone (III) with molecular bromine or N-bromosuccinimide gives *alpha*-

bromo-*gamma*-butyrolactone (XIII) which upon reaction with an imidazole or triazole provides the desired product. This synthetic route is shown in the following reaction scheme G.

Reaction Scheme G

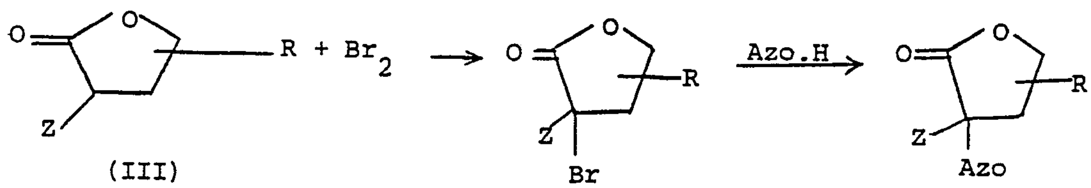


(III) (XIII)

Reference is made to U.K. Patent Specification Nos. 1,530,172 and 1,601,423 cited earlier, for guidance regarding the preparation of the acid addition salts and metal salt complexes.

The following examples, in illustrating methods for preparing compounds of Formula II, also illustrate the preparation, according to the invention, of compounds of Formula III.

Example 1

Preparation of 4-ethyl-2-phenyl-2-(1-imidazolyl methyl)-*gamma*-butyrolactone (Compound 2, Table I)

A. 4-ethyl-2-phenyl-*gamma*-butyrolactone

To a mixture of 117 g (1.0 mol) benzyl cyanide, 72 g (1.1 mol) powdered 86% potassium hydroxide and 250 ml. dimethylformamide under a nitrogen atmosphere there is added dropwise 74 g (1.0 mol) of 1,2-epoxy-butane at 5°C. The temperature is held at 5°C for 30 minutes and allowed to warm to room temperature. The reaction mixture is poured into 1,500 ml water and extracted with five 200 ml portions of toluene. To the combined toluene extracts is added 50 ml of 50% by weight hydrochloric acid and the mixture is heated on a steam bath for one hour. The toluene layer is then water washed until it is neutral, dried over sodium sulfate and filtered. The solvent is removed under vacuum to give 135 g of a pale yellow oil.

B. 2-bromomethyl-4-ethyl-2-phenyl-*gamma*-butyrolactone

To a solution of 19.4 g (0.19 mol) diisopropylamine in 100 ml of dry tetrahydrofuran is added dropwise 12.3 g (0.19 mol) n-butyl lithium at −75°C under nitrogen. Stirring is continued for 30 minutes and then a solution of 30 g (0.16 mol) of 4-ethyl-2-phenyl-*gamma*-butyrolactone in 50 ml tetraydrofuran is added dropwise over a period of one hour. The resulting mixture is further stirred for 30 minutes and a mixture of 33 g (0.19 mol) dibromomethane and 34 g (0.19 mol) hexamethylphosphoramide is added dropwise. The reaction temperature is maintained at −75°C throughout the additions. The reaction mixture is stirred at room temperature overnight and is then poured into 500 ml of saturated ammonium chloride solution and extracted with ether. The combined ether extracts are dried over sodium sulfate and filtered. Solvent is removed under vacuum to give 39 g of an oil which is further purified by bulb-to-bulb vacuum distillation to give 26 g of pure product.

C. 4-ethyl-2-phenyl-2-(1-imidazolylmethyl)-*gamma*-butyrolactone

A mixture of 5 g (0.0177 mol) of 2-bromomethyl-4-ethyl-2-phenyl-*gamma*-butyrolactone, 10 g (0.15 mol) imidazole and 0.5 ml dimethyl sulfoxide is heated at 120°C for twenty hours. The crude product is then dissolved in methylene chloride and washed with water. Methylene chloride is evaporated and the crude product is purified by passing through a silica gel column. The impurities are removed with ether and the product is eluted with ethyl acetate. A total of 2.2 g of pure product is obtained.

nmr: ($CDCl_3$): δ 0.8 (t, 3H), 1.4 (m, 2H) 2.5 (m, 2H), 4.0 (m, 1H) 4.4 (q, 2H), 6.7—7.5 (m, 8H).

Example 2

Preparation of 2-(2,4-dichlorophenyl)-4-ethyl-2-(1-imidazolylmethyl)-*gamma*-butyrolactone (Compound 3, Table I)

A. 2-(2,4-dichlorophenyl)-4-ethyl-*gamma*-butyrolactone

To a mixture of 186 g (1 mol) of 2,4-dichlorobenzyl cyanide and 60 g (1.1 mol) of powdered potassium hydroxide in 400 ml of dry dimethyl formamide there is added dropwise 75 g (1 mol) of 1,2-epoxy butane at 10°C. The resulting brown reaction mixture is stirred at room temperature overnight. It is then poured into water and extracted with ether. The combined ether extracts are washed with water, 5% by weight sodium chloride solution and dried over magnesium sulfate. Solvent is evaporated to give 228 g of a brown oil which is assigned the iminolactone structure due to strong ir absorption at 1680 $cm^{-1}$. This material is redissolved in toluene and 300 ml 25% by weight hydrochloric acid are added. The mixture is heated over a steam bath for two hours. The organic layer is separated and washed with water, saturated sodium chloride solution and dried over magnesium sulfate. Solvent is evaporated to give a light yellow oil which

is further purified by vacuum distillation (150°C/0.2 mm) to give 200 g of desired product.

ir ($CHCl_3$): 1780 $cm^{-1}$

nmr ($CDCl_3$): δ 1.0 (t, 3H), 1.3—30 (m, 4H), 4.5 (m, 2H), 7.1—7.6 (m, 3H).

B. 2-bromomethyl-4-ethyl-2-(2,4-dichlorophenyl)-*gamma*-butyrolactone

To a solution of 14.8 g (0.23 mol) diisopropylamine in 100 ml of dry tetrahydrofuran there is added 14.8 g (0.23 mol) of *n*-butyllithiumn at −60°C under nitrogen. Stirring is continued for 15 minutes and then there is added dropwise over a period of one hour a solution of 50 g (0.19 mol) 2-(2,4-dichlorophenyl)-4-ethyl-*gamma*-butyrolactone in 75 ml of tetrahydrofuran is added dropwise in one hour. The resulting mixture is further stirred for an additional 30 minutes and then the temperature is raised to −40°C and a solution of 40.3 g (0.23 mol) dibromomethane and 41.5 g (0.23 mol) of hexamethylphosphoramide is added dropwise over a period of 45 minutes. The reaction mixture is stirred at room temperature overnight and it is then poured into 500 ml of saturated ammonium chloride solution and extracted with ether. The combined ether extracts are dried over sodium sulfate and filtered. The solvent is removed under vacuum and the residue is further purified by vacuum distillation (158°C/0.1 mm) to give 47 g of a yellow oil.

C. 2-(2,4-dichlorophenyl)-4-ethyl-2-(1-imidazolylmethyl)-*gamma*-butyrolactone

A mixture of 9 g (0.03 mol) of 2-bromomethyl-4-ethyl-2-(2,4-dichlorophenyl)-*gamma*-butyrolactone, 10 g (0.15 mol) imidazole and 1 ml dimethyl sulfoxide is heated at 130°C for 3 days. The crude product is then dissolved in methylene chloride and washed with water. The product is further purified by converting to its hydrochloride salt and then back-neutralizing with diluted ammonium hydroxide solution to give 3 g of pure desired product.

nmr ($CDCL_3$): δ 0.8 (t, 3H), 1.4 (m, 2H), 2.5 (m, 2H), 3.6 (m, 1H), 4.6 (q, 2H), 6.9—7.7 (m, 6H).

## Examples 3—14

Following the procedures given in the above Examples 1 and 2 the additional compounds are prepared:

(3) 3 and/or 4-*n*-butyl-2-phenyl-2-[1-(1,2,4-triazolyl)methyl]-*gamma*-butyrolactone
(4) 3 and/or 4-*n*-butyl-2-phenyl-2-[4-(1-1,2,4-triazolyl)methyl]-*gamma*-butyrolactone
(5) 3 and/or 4-*n*-butyl-2-(4-chlorophenyl)-2-[1-(1,2,4-triazolyl)methyl]-*gamma*-butyrolactone
(6) 3 and/or 4-ethyl-2-(4-chlorophenyl)-2-[1-(1,2,4-triazolyl)methyl]-*gamma*-butyrolactone
(7) 3 and/or 4-ethyl-2-(4-methylphenyl)-2-[1-(1,2,4-triazolyl)methyl]-*gamma*-butyrolactone
(8) 3 and/or 4-*n*-octyl-2-(2,4-dichlorophenyl)-2-[1-(1,2,4-triazolyl)methyl]-*gamma*-butyrolactone
(9) 3 and/or 4-ethyl-2-(2-methoxyphenyl)-2-[1-(1,2,4-triazolyl)methyl]-*gamma*-butyrolactone
(10) 3 and/or 4-ethyl-2-phenyl-2-[1-(1,2,4-triazolyl)]-*gamma*-butyrolactone

When a 1-H-1,2,4-triazole free base is used as a starting material and there results a mixture of 1-substituted and 4-substituted 1,2,4-triazoles, these triazoles can usually be separated by conventional chemical separation techniques such as extraction, chromatography or recrystallization.

Table I below gives the structure of the compounds prepared by foregoing Examples 1 and 2 and one additional compound (Compound 2a) which was prepared by following the procedures of Examples 1 and 2. Table II gives elemental analyses or nmr data for Compounds Nos. 1, 2 and 2a listed in Table I.

TABLE I

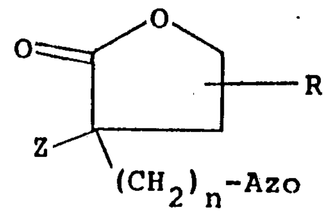

$(CH_2)_n$-Azo (acid additon salt or metal complex)

| Compound No. | Z | R | n | Azo | Acid addition salt or metal salt complex |
|---|---|---|---|---|---|
| 1 | $C_6H_5$ | 4-$C_2H_5$ | 1 | 1-Imidazolyl | - |
| 2 | 2,4-$Cl_2C_6H_3$ | 4-$C_2H_5$ | 1 | 1-Imidazolyl | - |
| 2a | 2,4-$Cl_2C_6H_3$ | 4-$C_4H_9$-*n* | 1 | 1-(1,2,4-Triazolyl) | - |

TABLE II

| Compound No. | MP (°C.) | Elemental Analysis: Cal'd (Found) C | H | Cl | N | O |
|---|---|---|---|---|---|---|
| 1 | Oil | 71.09<br>(71.25) | 6.71<br>(6.85) | -<br>- | 10.36<br>(10.75) | 11.84<br>(12.15) |
| 2 | Oil | 56.65<br>(56.47) | 4.75<br>(5.09) | 20.90<br>(20.31) | 8.26<br>(8.87) | 9.43 |
| 2a | Oil | identified by nmr and mass spectra, m/e = 368 | | | | |

nmr ($CDCl_3$): $\delta$ 1.1 (m, 9H),
2.3 (q, 1H), 3.4 (m, 2H),
4.9 (q, 2H), 7.6 (m, 4H),
8.1 (s, 4H).

## Claims

1. A process for synthesizing a compound of the formula

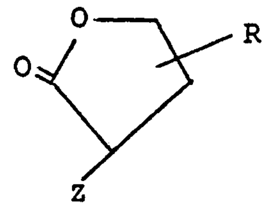

which comprises (1) reacting, under basic conditions and at a temperature of 5°C to room temperature, an arylacetonitrile of the formula

$$Z—CH_2CN$$

with an epoxide of the formula

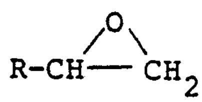

in the presence of basic hydroxide and organic solvent, and (2) hydrolyzing the resulting *gamma*-iminobutyrolactone produced thereby to yield the desired compound, wherein Z is phenyl or naphthyl each optionally substituted with up to 3 of the same or different substituents selected from (1) halo, (2) nitro, (3) trihalomethyl, (4) cyano, (5) ($C_1$—$C_4$)alkyl, (6) ($C_1$—$C_4$)alkoxy, (7) ($C_1$—$C_4$)alkylthio, (8) ($C_1$—$C_4$)alkylsulfinyl, (9) ($C_1$—$C_4$)alkylsulfonyl, (10) phenyl, (11) benzyl, (12) phenoxy, (13) phenylthio, (14) phenylsulfinyl, (15) phenylsulfonyl and (16) groups (10) to (15) above which contain up to 3 ring substituents selected from groups (1) to (9) above, R is H, ($C_1$—$C_{10}$)alkyl, ($C_3$—$C_8$)alkenyl, ($C_3$—$C_8$)alkynyl, phenyl, naphthyl, phenyl($C_1$—$C_4$)alkyl, naphthyl($C_1$—$C_4$)alkyl or each of the last four groups containing up to 3 the same or different ring substituents selected from groups (1) to (16) listed above in connection with the definition of Z.

2. A process as claimed in Claim 1, wherein Z is phenyl or phenyl substituted with up to two halogen atoms and; R is ($C_1$ to $C_4$)alkyl.

3. A process as claimed in Claim 2, wherein Z is phenyl or 2,4-dichlorophenyl and R is methyl, or *n*-butyl.

4. A process as claimed in Claim 2, wherein (1) Z is phenyl and R is 3- and 4-ethyl, (2) Z is phenyl and R is 4-ethyl, or (3) Z is 2,4-dichlorophenyl and R is ethyl or *n*-butyl.

5. A process as claimed in Claim 1, wherein Z is phenyl or phenyl substituted with up to two chlorine atoms, a methyl group or a methoxy group and R is hydrogen, methyl, ethyl, *n*-butyl, *n*-octyl or phenyl.

6. A process as claimed in Claim 5, wherein (1) Z is phenyl and R is *n*-butyl, (2) Z is 4-chlorophenyl and R is ethyl or *n*-butyl, (3) Z is 2,4-dichlorophenyl and R is *n*-octyl or phenyl, (4) Z is 2-methoxyphenyl and R is ethyl, (5) Z is 2,4-dichlorophenyl and R is hydrogen.

## Patentansprüche

1. Verfahren zum Synthetisieren einer Verbindung der Formel

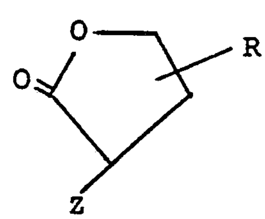

welches darin besteht, (1) unter basischen Bedingungen und einer Temperatur von 5°C bis Zimmertemperatur ein Arylacetonitril der Formel

$$Z—CH_2CN$$

mit einem Epoxid der Formel

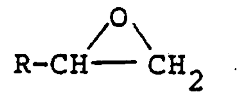

in Gegenwart eines basischen Hydroxids und eines organischen Lösungsmittels umzusetzen und (2) das erhaltene Gammaiminobutyrolacton zur Gewinnung der gewünschten Verbindung zu Hydrolisieren, wobei Z ein Phenyl oder Naphtyl, jeweils gegebenenfalls substituiert mit bis zu 3 gleichen oder verschiedenen Substituenten, ausgewählt aus (1) Halogen, (2) Nitro, (3) Trihalogenmethyl, (4) Cyano, (5) $(C_1—C_4)$Alkyl, (6) $(C_1—C_4)$Alkoxy, (7) $(C_1—C_4)$Alkylthio, (8) $(C_1—C_4)$Alkylsulfinyl, (9) $(C_1—C_4)$Alkylsulfonyl, (10) Phenyl, (11) Benzyl, (12) Phenoxy, (13) Phenylthio, (14) Phenylsulfinyl, (15) Phenylsulfonyl und (16) vorstehenden Gruppen (10) bis (15), die bis zu 3 Ringsubstituenten enthalten, ausgewählt aus den vorstehenden Gruppen (1) bis (9), R H, $(C_1—C_{10})$Alkyl, $(C_3—C_8)$Alkenyl, $(C_3—C_8)$Alkinyl, Phenyl, Naphthyl, Phenyl$(C_1—C_4)$alkyl, Naphtyl$(C_1—C_4)$alkyl oder jede der letzten vier Gruppen ist, die bis zu 3 gleichen oder verschiedenen Ringsubstituenten aufweist, ausgewählt aus den vorstehend im Zusammenhang mit der Definition Z aufgeführten Gruppen (1) bis (16).

2. Verfahren nach Anspruch 1, wobei Z Phenyl oder Phenyl, substituiert mit bis zu zwei Halogenatomen, is und R $(C_1—C_4)$Alkyl bedeutet.

3. Verfahren nach Anspruch 2, worin Z phenyl oder 2,4-Dichlorphenyl ist und R Methyl oder n-Butyl ist.

4. Verfahren nach Anspruch 2, wobei (1) Z Phenyl ist und R 3- und 4-Ethyl bedeutet, (2) Z Phenyl ist und R 4-Ethyl darstellt oder (3) Z 2,4-Dichlorphenyl ist und R Ethyl oder n-Butyl bedeutet.

5. Verfahren nach Anspruch 1, wobei Z Phenyl oder Phenyl, substituiert mit bis zu zwei Chloratomen, einer Methylgruppe oder einer Methoxygruppe, ist und R Wasserstoff, Methyl, Ethyl, n-Butyl, n-Octyl oder Phenyl bedeutet.

6. Verfahren nach Anspruch 5, wobei (1) Z Phenyl ist und R n-Butyl bedeutet, (2) Z 4-Chlorphenyl ist und R Ethyl oder n-Butyl darstellt, (3) Z 2,4-Dichlorphenyl ist und R n-Octyl oder Phenyl ist, (4) Z 2-Methoxyphenyl ist und R Ethyl bedeutet, (5) Z 2,4-Dichlorphenyl ist und R für Wasserstoff steht.

**Revendications**

1. Un procédé pour synthétiser un composé de formule

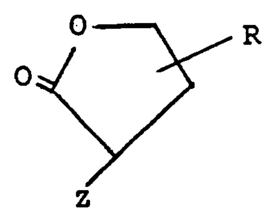

qui consiste: (1) à faire réagir, dans des conditions basiques et à une température comprise entre 5°C et la température ambiante, un arylacétonitrile ayant la formule

$$Z—CH_2CN$$

avec un époxyde, ayant la formule

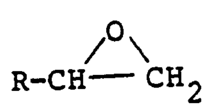

en présence d'un hydroxyde basique et d'un solvant organique, et (2) à hydrolyser la *gamma*-iminobutyrolactone résultante ainsi produite, en obtenant ainsi le composé désiré, formules dans lesquelles Z est un radical phényl ou naphthyl, chacun pouvant être éventuellement substitué par au plus 3 substituants, identiques ou différents, choisis parmi (1) halo, (2) nitro, (3) trihalométhyl, (4) cyano, (5) $(C_1—C_4)$-alcoyl, (6) $(C_1—C_4)$-alcoyloxy, (7) $(C_1—C_4)$-alcoylthio, (8) $(C_1—C_4)$-alcoylsulfinyl, (9) $(C_1—C_4)$-alcoylsulfonyl, (10) phényl, (11) benzyl, (12) phénoxy, (13) phénylthio, (14) phénylsulfinyl, (15) phénylsulfonyl et (16) les groupes (10) à (15) ci-dessus contenant jusqu'à 3 substituants sur le noyau choisis dans les groupes (1) à (9) ci-dessus, R est H, $(C_1—C_{10})$alcoyl, $(C_3—C_8)$alcényl, $(C_3—C_8)$alcoynyl, phényl, naphthyl, phényl$(C_1—C_4)$alcoyl, naphthyl$(C_1—C_4)$alcoyl ou chacun des quatre derniers groupes contenant

jusqu'à 3 subsituants sur le noyau, identiques ou différents, choisis dans les groupes (1) à (16) énumérés ci-dessus relativement à la définition de Z.

2. Un procédé comme revendiqué dans la revendication 1, dans lequel Z est un phényl ou un phényl substitué avec au plus deux atomes d'halogène et R est ($C_1$ à $C_4$)alcoyl.

3. Un procédé comme revendiqué dans la revendication 2, dans lequel Z est phényl ou 2,4-dichlorophényl et R est méthyl ou *n*-butyl.

4. Un procédé comme revendiqué dans la revendication 2, dans lequel (1) Z est phényl et R est 3- et 4-éthyl, (2) Z est phényl et R est 4-éthyl, ou (3) Z est 2,4-dichlorophényl et R est éthyl ou n-butyl.

5. Un procédé comme revendiqué dans la revendication 1, dans lequel Z est phényl ou phényl substituté par au plus deux atomes de chlore, un groupe méthyl ou un groupe méthoxy et R est hydrogène, méthyl, éthyl, *n*-butyl, *n*-octyl ou phényl.

6. Un procédé comme revendiqué dans la revendication 5, dans lequel (1) Z est phényl et R est *n*-butyl, (2) Z est 4-chlorophényl et R est éthyl ou *n*-butyl, (3) Z est 2,4-dichlorophényl et R est *n*-octyl ou phényl, (4) Z est 2-méthoxyphényl et R est éthyl, (5) Z est 2,4-dichlorophényl et R est l'hydrogène.